(19) Europäisches Patentamt
European Patent Office
Office européen des brevets

(11) Publication number : 0 490 564 A1

(12) **EUROPEAN PATENT APPLICATION**

(21) Application number : 91311268.6

(51) Int. Cl.[5] : C07D 275/03

(22) Date of filing : 04.12.91

(30) Priority : 11.12.90 US 626122

(43) Date of publication of application : 17.06.92 Bulletin 92/25

(84) Designated Contracting States : AT BE CH DE DK ES FR GB GR IT LI LU NL SE

(71) Applicant : ROHM AND HAAS COMPANY
Independence Mall West
Philadelphia Pennsylvania 19105 (US)

(72) Inventor : Petigara, Ramesh Balubhai
2185 Stewart Drive
Hatfield, Pennsylvania 19440 (US)
Inventor : Horton, Isaac Byron
A102 Sommerset House Condo
Fort Washington, Pennsylvania 19034 (US)

(74) Representative : Smith, Julian Philip Howard et al
Rohm and Haas (UK) Limited, European Operations Patent Dept., Lennig House, 2 Masons Avenue
Croydon CR9 3NB (GB)

(54) **Process for preparing 3-isothiazolone compounds.**

(57) A process for the prepartion of salt-free, and optionally water-free, isothiazolones comprising treating a solution of an acid salt of an isothiazolone with a basic anion exchange resin, perferably a weak base anion exchange resin, and separating the free base isothiazolone solution from the spent resin is disclosed. The resultant salt-free isothiazolones are useful as microbicides in environments where even low concentrations of metal salts are undesirable, for example, latex emulsions and corrosion sensitive aqueous systems.

EP 0 490 564 A1

This invention concerns the manufacture of 3-isothiazolones, and in particular it is concerned with a process for preparing 3-isothiazolones which are free of metal salts.

The 3-isothiazolones to which this invention relates have the general formula

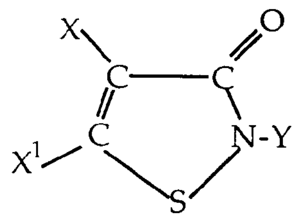

wherein Y is an unsubstituted or substituted alkyl of 1 to 10 carbon atoms; an unsubstituted or halogen substituted alkenyl or alkynyl of 2 to 10 carbon atoms; an aralkyl or halogen-, lower alkyl-, or lower alkoxy-substituted aralkyl of up to 10 carbon atoms; X is hydrogen or a $(C_1-C_2)$alkyl; and $X^1$ is hydrogen, chlorine, or a $(C_1 - C_2)$alkyl. These isothiazolone are disclosed in U.S. Patents 3,523,121 and 3,761,488.

These 3-isothiazolones are well known as microbicides and are employed in many industrial and household systems. Since certain 3-isothiazolones in aqueous solutions are generally unstable, stabilizing divalent metal salts as described in U.S.-A-3,870,795 and 4,087,878 are usually incorporated. In certain applications, e.g., preservation of latex emulsion, these metal stabilization salts cause gelation problems which can reduce the performance or value of such latex emulsions.

Another problem with such metal stabilization salts is that they cause corrosion in certain systems. For example, chloride salts have a corrosive effect on many metals and are to be avoided where possible. In water treatment systems where low cation and anion levels are important, it is desirable to eliminate such salts. In the stabilization of plastic articles, salts may contribute to deterioration of optical properties and/or increase water pickup and haze levels.

In some cosmetic formulations, it is also important to eliminate inorganic salt, especially nitrate.

Isothiazolone hydrochloride salts (isothiazolone•HCl) are generated in the general prior art process for manufacturing isothiazolones. Such a process for the manufacture of a mixture of 5-chloro-2-methyl-3-isothiazolone and 2-methyl-3-isothiazolone is described in U.S.-A-3,849,430 and 4,939,266. The isothiazolone•HCl is generated in the chlorination/cyclization step of this process during which either a di-(or tri)thiodiamide or a mercaptoamide is cyclized:

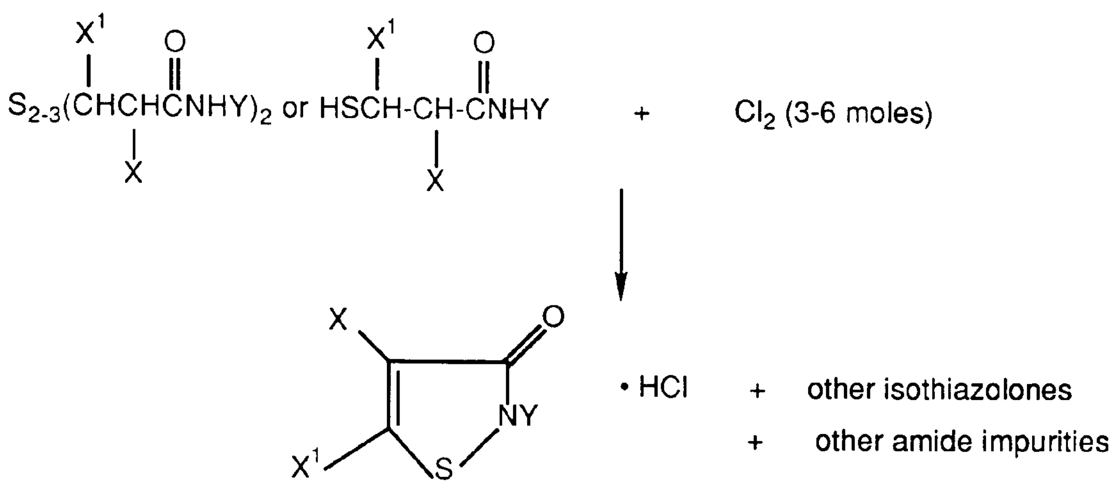


The chlorination slurry is then filtered, and the isothiazolone•HCl cake is washed and reslurried or dissolved in the same or different solvent. In aqueous systems, a neutralizing agent such as sodium carbonate, magnesium oxide or calcium oxide is then added to yield the free base isothiazolone and a chloride salt (in this case the chloride salt remains with the free base isothiazolone in aqueous solution):

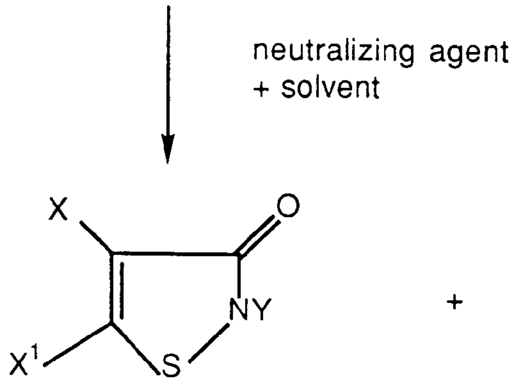


+ chloride salt

Certain organic amines have been suggested as neutralizing agents in non-aqueous organic media in U.S.-A-4,824,957. Such organic amines produce organic amine hydrohalide salts as byproducts. The amount of organic amine required to neutralize the isothiazolone hydrohalide salt is difficult to determine and thus the neutralization endpoint cannot be controlled precisely. Any excess organic amine remains in the organic solvent solution of the free base isothiazolone after neutralization and contaminates the final product solution, and furthermore may also chemically react with the free base isothiazolone to produce additional byproducts. In addition, these residual amines may also act as a source of nitrosamine contaminants, if such free base isothiazolone were to be formulated to aqueous solutions stabilized with nitrate salts.

The free base isothiazolone and amine hydrochloride salt (amine•HCl) which are formed by the neutralization of isothiazolone•HCl with organic amines are separated by filtering off the solid amine•HCl salt from the solution of free base isothiazolone. However, the amine•HCl is sparingly to appreciably soluble in the solvent, and consequently the final isothiazolone product may not be entirely salt-free.

None of the prior art processes produces an isothiazolone which is substantially pure, salt-free, and water-free.

A copending patent application (US Serial No 464472, filed january 12, 1990) commonly assigned to the assignee of the present application teaches a process for producing salt-free isothiazolones by neutralizing isothiazolone acid salts with anhydrous ammonia, preferably in organic solvents, in order to separate (by filtration) the insoluble ammonium chloride neutralization salt from the solution of free base isothiazolone.

It is generally known in the field of ion exchange technology that it is possible to remove acidic impurities from solutions containing such acidic materials by treatment with basic anion exchange resins (JE Salmon and DK Hale, Ion Exchange, A Laboratory Manual, pp 9-10, 1959). For example, strong base anion exchange resins (resin A, where Ⓟ represents a crosslinked polymeric backbone) or weak base anion exchange resins (resin B) are capable of removing acidic materials, such as HCl, from aqueous solutions according to Equations I and II.

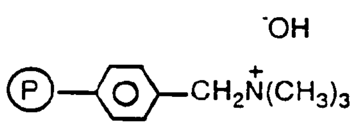

Resin A

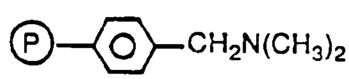

Resin B

Resin A + HCl 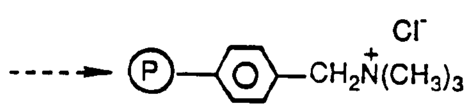 + $H_2O$ Equation I

Resin B + HCl 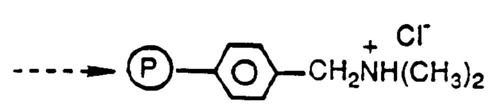 Equation II

Although the general use of basic anion exchange resins for deacidification/neutralization is well-known, the use of basic anion exchange resins for the neutralization of isothiazolone acid salts to their free base isothiazolone form has never been disclosed.

It is an object of the present invention to provide a process to produce salt free isothiazolones of high purity and in high yield, which are optionally also water free.

Accordingly in one aspect the invention provides a process for preparing a 3-isothiazolone of the formula

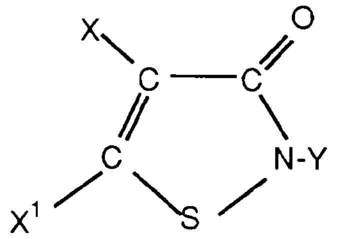

wherein

Y is an unsubstituted or hydroxy- or halogen-substituted alkyl of 1 to 10 carbon atoms; an unsubstituted or halogen-substituted alkenyl or alkynyl of 2 to 10 carbon atoms; or an aralkyl of up to 10 carbon atoms optionally substituted with halogen, lower alkyl, or lower alkoxy;

X is hydrogen or a ($C_1$-$C_2$) alkyl; and

$X^1$ is hydrogen, chlorine, or a ($C_1$-$C_2$) alkyl;

comprising

(a) preparing a solution of isothiazolone acid salt of the formula

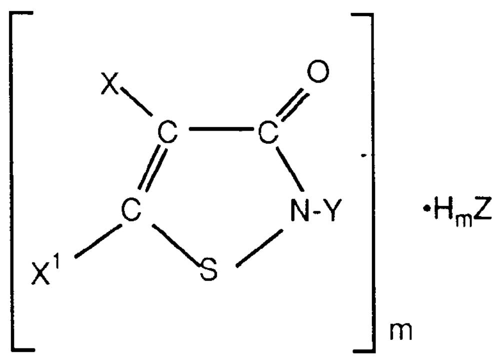

wherein

Z is chloride, bromide, sulfate, fluorosulfonate or trifluoromethyl sulfonate;

m is 1 when Z is chloride, bromide, fluorosulfonate, or trifluoromethyl sulfonate and m is 2 when Z is sulfate;

(b) treating said solution with a basic anion exchange resin; and

(c) separating the resultant free base isothiazolone solution from the resin.

In a preferred embodiment of the invention, weakly basic anion exchange resins are used to treat solutions of IHZ.

Y is preferably methyl, ethyl, propyl, isopropyl, butyl, hexyl, octyl, hydroxymethyl, chloromethyl, chloropropyl, benzyl, 4-methoxybenzyl, 4-chlorobenzyl, phenethyl, 2-(4-chlorophenyl)ethyl, 4-phenylbutyl, and the like.

Z is preferably chlorine or bromine, and most preferably chlorine.

The expressions "lower" alkyl, lower alkoxy, and the like mean that the alkyl or alkoxy portion thereof has about 1 to 2 carbon atoms.

The process of the present invention comprises essentially the steps of contacting a solution of IHZ with a basic anion exchange resin to neutralize the $H_mZ$ present to form free base isothiazolone, and having $H_mZ$ bound to the resin, then separating the free base isothiazolone solution from the spent resin. For example, when IHZ is isothiazolone•HCl, the neutralization reaction with a basic anion exchange resin, in this case a weak base resin, may be represented by Equation III:

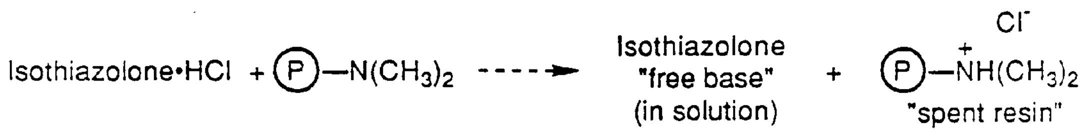


III

Since the free base isothiazolone is obtained in a solution free of any dissolved or suspended solids when the invention is practiced in the column mode, there is no separate filtration step necessary to remove insoluble or precipitated neutralization salts that characterize some of the prior art processes, e.g., the use of organic amines to produce insoluble amine hydrohalide salts (U.S.-A-4,824,957).

In addition to water, preferred organic solvents for dissolving the IHZ are selected from the group consisting of $(C_1\text{-}C_5)$alkyl alcohol and $(C_2\text{-}C_4)$alkylene glycol. The organic solvents are selected such that the both the IHZ and free base isothiazolone are essentially soluble.

A particularly preferred group of solvents is selected from the group consisting of methanol, ethanol, isoproponal, butanol, ethylene glycol, propylene glycol, 1,3- or 1,4-butanediol, dipropylene glycol and diethylene glycol.

Other strong acid salts such as the (isothiazolone)$_2$•$(H_2SO_4)$ or (isothiazolone) • $HSO_3F$ or (isothiazolone) • $HSO_3CF_3$ salts may also be neutralized with a basic anion exchange resin to produce the free base isothiazolone.

Although both strong base and weak base anion exchange resins may be suitable for the neutralization of IHZ to give the free base isothiazolone solution, we have found that weak base anion exchange resins are preferred for producing salt-free isothiazolones of high purity in high yield. Strong base anion exchange resins, i.e, those containing a quaternary ammonium hydroxide structure (Resin A, supra), such as Amberlite® IRA-420C-OH and Amberlite® IRA-900C-OH anion exchange resins, may degrade certain free base isothiazolones partly during the neutralization step and partly during the subsequent solvent removal step. In general, when strongly basic anion exchange resins are used (see Examples 13-20), the free base isothiazolones are obtained with lower recovery yields/purity and poorer colour quality of the unstripped isothiazolone solution compared to when weak base anion exchange resins are used.

In addition to the strong base anion exchange resins, a wide range of weak base anion exchange resins are suitable for neutralizing IHZ without adversely affecting the quality and purity of the free base isothiazolone. Suitable weak base anion exchange resins may be characterized by the structures for Resins C and D (infra), where Ⓟ represents a crosslinked polymeric backbone.

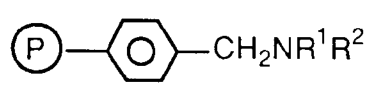

Resin C

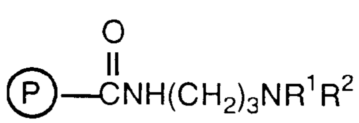

Resin D

$R^1$ and $R^2$ may be independently selected from the group consisting of hydrogen, $(C_1\text{-}C_4)$alkyl, $(CH_2CH_2NH)_nH$ where n = 1-3, and β-hydroxy$(C_1\text{-}C_4)$alkyl; preferably $R^1$ and $R^2$ are methyl. In addition, partially oxidized analogues of Resins C and D are suitable for neutralizing IHZ; in this case, the weak base anion exchange resin may contain from 5-25% of the corresponding amine oxide form (Resin E).

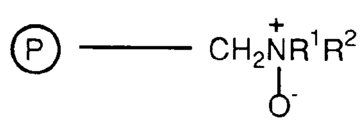

Resin E

Weak base anion exchange resins suitable for neutralizing IHZ to produce salt-free isothiazolones of high purity in high yields may be further characterized in that the weakly basic functional groups of Resins C, D and E possess a pKb (relative to water) of approximately 3 to about 7, preferably from about 3.5 to about 4.5. The

pKb value is a measure of basicity, with lower values representing stronger basicity.

These types of resin are well-known and are available commercially, for example from Rohm and Haas Company, as Amberlit® IRA-93, Amberlite® IRA-35, Amberlite® IRA-68, Amberlit® IRA-94, Duolite® A-368 and Amberlyst® A21, or from Mitsubishi Kasei, as Diaion® WA-30 ion exchange resins.

The following examples illustrate the process of the present invention; they are illustrative only, and are not intended to limit the scope of the invention.

EXAMPLES

General Procedure

A variety of ion exchange resins was evaluated for the neutralization of isothiazolone•HCl salts. Each resin was first conditioned in the following manner. A 1-liter neutralization reaction vessel (3-necked flask fitted with a pH meter, stirrer, a bottom fritted disk and a take-off valve) was charged with approximately 300 ml of the ion exchange resin to be evaluated. The resin was first washed with 200 ml distilled water (twice), treated with 20 ml of concentrated HCl in 200 ml of water and then washed twice with 200 ml of distilled water. The resin was then treated with 42 g of 50% NaOH in 200 ml of water and finally washed four times with distilled water to a final slurry pH of approximately 10. If methanol was to be the solvent for the neutralization step, the resin was prewashed with 300 ml of methanol and finally slurried with 250 ml of fresh methanol. If water was to be the solvent for the neutralization step, the resin was slurried with 250 ml of distilled water.

1. Batch Mode

Method 1A

A solution of the isothiazolone•HCl salt (approximately 33% by weight in approximately 100 ml solution) in distilled water (or methanol) was pumped into the 1-liter neutralization vessel containing the conditioned ion exchange resin to be evaluated. The initial pH of the ion exchange resin slurry was typically 10-11. After complete addition of the isothiazolone•HCl salt solution, the pH of the slurry decreased to 5-6. The mixture was then stirred for 15 minutes, after which the pH rose to 7.5. The liquid phase was then drained from the vessel through the fritted bottom disk by applying 3 psig pressure, then 200 ml of distilled water (or methanol) was added, followed by stirring for another 15 minutes and the liquid phase was again drained from the flask by applying 3 psig pressure. This washing step was repeated 3 times. The final pH of the slurry was approximately 7. All of the liquid washes were combined and analyzed for isothiazolone free base yield, purity and color quality. Color was an indication of any degradation caused by exposure to the basic conditions of the specific ion exhange resin environment: a free base isothiazolone solution having a yellow color indicated no degradation (good quality), an amber color indicated moderate degradation, and a dark amber color indicated significant degradation (very poor quality). When a low-boiling alcohol such as methanol, ethanol or propanol was used, the effluents (washes) were combined and stripped under reduced pressure (45°C/20 -->1 mm Hg) to obtain the free base isothiazolones in pure form (free of solvent).

Method 1B

Alternatively, the batch mode experiment was carried out in a 1-liter vessel followed by isolation of the ion exchange resin by filtration on a Buchner funnel. In this case the ion exchange resin was thoroughly washed with distilled water (or methanol) and the resin washings/filtrates were combined (stripped when methanol was used) and subjected to analysis as described above.

2. Column Mode (Method 2)

The conditioned ion exchange resin was placed in a 3X68 cm glass column fitted with a fritted bottom. A solution of the isothiazolone•HCl salt (approximately 33% by weight in approximately 100 ml solution) in distilled water (or methanol) was pumped into the column at a rate of 5.5 ml/minute. Upon complete addition of the isothiazolone•HCl salt solution, additional distilled water (or methanol) was pumped through the column corresponding to 4 bed volumes. The effluents were combined and analyzed for isothiazolone yield, purity and color quality. When a low-boiling alcohol such as methanol, ethanol or propanol was used, the effluents (washes) were combined and stripped under reduced pressure (45C/20 -->1 mm Hg) to obtain the free base isothiazolones in pure form (free of solvent).

3. Ion Exchange resins Evaluated

Five different ion exchange resins were evaluated: two strong base anion exchange resins (Amberlite® IRA-900C-OH (macroreticular styrenic resin in hydroxide form) and Amberlite® IRA-420C-OH (gel styrenic resin in hydroxide form) and 3 weak base anion exchange resins (Amberlite® IRA-68 (gel acrylic resin), Amberlyst® A21 (macroreticular styrenic resin), and Diaion® WA-30 (macroporous styrenic resin)). The Amberlite® and Amberlyst® ion exchange resins were supplied by Rohm and Haas Co.; the Diaion® resin was supplied by Mitsubishi Kasei.

Example 1

Neutralization of 75/25 Mixture of 5-Chloro-2-Methyl-3-Isothiazolone and 2-Methyl-3-Isothiazolone Using a Weak Base Ion-Exchange resin in Methanol

This example illustrates using ethyl acetate as the organic solvent for the chlorination/cyclization step which is carried out following the process of U.S. Patents 3,849,430 and 4,939,266.

Step 1: Chlorination of N-Methyl-3-Mercaptopropionamide (MMPA)

A 1-liter jacketed kettle with a bottom take-off was equipped with an overhead agitator, a thermometer, a chlorine inlet, an inlet for a solution of MMPA in ethyl acetate, and a condenser connected to a caustic scrubber. To this kettle was charged ethyl acetate. To the heel of ethyl acetate were co-fed chlorine and the MMPA solution over a 1-3 hour period while maintaining good mixing and the temperature at 27±2°C. The resultant chlorination slurry of isothiazolone•HCl salts was filtered to isolate the isothiazolone•HCl cake. The isothiazolone•HCl cake was washed thoroughly with ethyl acetate to yield a highly pure mixture of isothiazolone•HCl salts (99.9% after drying, by HPLC and HCl analyses, 73.5 % 5-chloro-2-methyl-3-isothiazolone/26.5% 2-methyl-3-isothiazolone or 2.8:1 ratio).

Step 2: Neutralization of the Isothiazolone•HCl Salt

Amberlite® IRA-68 ion exchange resin was used to neutralize the isothiazolone•HCl salt solution in methanol using the batch mode described in general procedure/method 1B. The resin washings and the filtrate were combined and stripped at 45°C/20 mm Hg to give 23.4 g of an off-white product. HPLC analysis indicated 98.6% purity for the solvent-stripped 5-chloro-2-methyl-3-isothiazolone/ 2-methyl-3-isothiazolone in an unchanged ratio of 2.7:1. Recovery/yield of the isothiazolone mixture was 98%. Overall yield was 84 mole percent based on starting MMPA.

Step 3: Formulation of 5-Chloro-2-methyl-3-isothiazolone/ 2-Methyl-3-isothiazolone in Dipropylene Glycol (DPG)

The above highly pure free base isothiazolone mixture was dissolved in an appropriate amount of DPG to give 5% isothiazolone (active ingredient) in DPG solution.

The formulation containing 5% active ingredient was then added to various latex emulsions at 30-100 ppm (active ingredient) providing excellent protection against biological fouling without causing any gel formation in the latex.

Example 2

Neutralization of 70/30 Mixture of 5-Chloro-2-Methyl-3-Isothiazolone and 2-Methyl-3-Isothiazolone Mixture Using a Weak Base Ion-Exchange Resin in Water

A 30 g sample of the isothiazolone•HCl mixture obtained according to Step 1 and Example 1, was dissolved in 55 ml of water. Amberlite® IRA-68 ion exchange resin was used to neutralize the isothiazolone•HCl salt in water using the batch mode described in general procedure/method 1B. After washing the spent resin with water in a plug-flow manner, the resulting 285 g of solution (pH 7.2) contained 5.7% 5-chloro-2-methyl-3-isothiazalone and 2.5% 2-methyl-3-isothiazolone (unchanged 70/30 ratio), corresponding to a recovery yield of 97.5%.

Example 3

Neutralization of 2-Methyl-3-Isothiazolone Using a Weak Base Ion Exchange Resin in Water

In a manner similar to step 1 of Example 1, an isothiazolone•HCl salt was obtained having purity of 98.2% in terms of 2-methyl-3-isothiazolone•HCl with a small amount (<1%) of 5-chloro-2-methyl-3-isothiazolone•HCl salt.

Amberlite® IRA-68 ion exchange resin was used to neutralize the isothiazolone•HCl salt solution in water using the batch mode described in general procedure/method 1A. The combined washes contained 99% of the theoretically recoverable free base 2-methyl-3-isothiazolone (900 g of an off-white solution with 3.94% by weight of isothiazolone).

Regeneration of the spent Amberlite® IRA-68 ion exchange resin was accomplished by treating the resin with 42 g of 50% NaOH in 200 ml of water, followed by draining the liquid phase by pressurizing the vessel, and then washing the resin 4 times with 200 ml of deionized water until the final pH of the resin slurry was approximately 10. The ion exchange resin was then suitable for reuse in subsequent neutralizations.

Example 4

Neutralization of 75/25 Mixture of 5-Chloro-2-Methyl-3-Isothiazolone and 2-Methyl-3-Isothiazolone Using a Weak Base Ion Exchange Resin in Water

In a manner similar to Step 1 of Example 1, an isothiazolone•HCl salt was obtained and subjected to neutralization. Amberlite® IRA-68 ion exchange resin was used to neutralize the isothiazolone•HCl salt solution in water using the batch mode described in general procedure/method 1A. The yield of isothiazolone free base was 98%, the isothiazolone mixture ratio remained unchanged at 75/25 and the color quality was very good (yellow).

Example 5

Neutralization of 75/25 Mixture of 5-Chloro-2-Methyl-3-Isothiazolone and 2-Methyl-3-Isothiazolone Using a Weak Base Ion Exchange Resin in Methanol

In a manner similar to Step 1 of Example 1, an isothiazolone•HCl salt was obtained and subjected to neutralization. Amberlite® IRA-68 ion exchange resin was used to neutralize the isothiazolone•HCl salt solution in methanol using the batch mode described in general procedure/method 1A. The yield of isothiazolone free base was 99%, the isothiazolone mixture ratio remained unchanged at 75/25 and the color quality was very good (yellow).

Example 6

Neutralization of 75/25 Mixture of 5-Chloro-2-Methyl-3-Isothiazolone and 2-Methyl-3-Isothiazolone Usine a Weak Base Ion Exchange Resin in Water

In a manner similar to Step 1 of Example 1, an isothiazolone•HCl salt was obtained and subjected to neutralization. Amberlite® IRA-68 ion exchange resin was used to neutralize the isothiazolone•HCl salt solution in water using the column mode described in general procedure/method 2. The yield of isothiazolone free base was 88-92%, the isothiazolone mixture ratio remained unchanged and the color quality was somewhat poor (amber).

Example 7

Neutralization of 75/25 Mixture of 5-Chloro-2-Methyl-3-Isothiazolone and 2-Methyl-3-Isothiazolone Using a Weak Base Ion Exchange Resin in Methanol

In a manner similar to Step 1 of Example 1, an isothiazolone•HCl salt was obtained and subjected to neutralization. Amberlite® IRA-68 ion exchange resin was used to neutralize the isothiazolone•HCl salt solution in methanol using the column mode described in general procedure/method 2. The yield of isothiazolone free base was 96%, the isothiazolone mixture ratio remained unchanged at 75/25 and the color quality of the free base

isothiazolone methanol effluent was very good (yellow).

Example 8

Neutralization ot 75/25 Mixture of 5-Chloro-2-Methyl-3-Isothiazolone and 2-Methyl-3-Isothiazolone Using a Weak Base Ion Exchange Resin in Water

In a manner similar to Step 1 of Example 1, an isothiazolone•HCl salt was obtained and subjected to neutralization. Amberlyst® A21 ion exchange resin was used to neutralize the isothiazolone•HCl salt solution in water using the batch mode described in general procedure/method 1A. The yield of isothiazolone free base in the aqueous effluent was 87%, the isothiazolone mixture ratio remained unchanged at 75/25 and the color quality was good (amber).

Example 9

Neutralization of 75/25 Mixture of 5-Chloro-2-Methyl-3-Isothiazolone and 2-Methyl-3-Isothiazolone Using a Weak Base Ion Exchange Resin in Methanol

In a manner similar to Step 1 of Example 1, an isothiazolone•HCl salt was obtained and subjected to neutralization. Amberlyst® A21 ion exchange resin was used to neutralize the isothiazolone•HCl salt solution in methanol using the batch mode described in general procedure/method 1A. The yield of solvent-stripped isothiazolone free base was 98%, the isothiazolone mixture ratio remained unchanged at 75/25, the purity was 96%, and the color quality was very good (yellow).

Example 10

Neutralization of 75/25 Mixture of 5-Chloro-2-Methyl-3-Isothiazolone and 2-Methyl-3-Isothiazolone Using a Weak Base Ion Exchange Resin in Water

In a manner similar to Step 1 of Example 1, an isothiazolone•HCl salt was obtained and subjected to neutralization. Amberlyst® A21 ion exchange resin was used to neutralize the isothiazolone•HCl salt solution in water using the column mode described in general procedure/method 2. The yield of isothiazolone free base was lower at 73%, the isothiazolone mixture ratio had decreased significantly and the color quality was very poor (dark amber).

Example 11

Neutralization of 75/25 Mixture of 5-ChLoro-2-Methyl-3-Isothiazolone and 2-Methyl-3-Isothiazolone Using a Weak Base Ion Exchange Resin in Methanol

In a manner similar to Step 1 of Example 1, an isothiazolone•HCl salt was obtained and subjected to neutralization. Amberlyst® A21 ion exchange resin was used to neutralize the isothiazolone•HCl salt solution in methanol using the column mode described in general procedure/method 2. The yield of isothiazolone free base was 97% with an unchanged isothiazolone mixture ratio and the color quality of the methanol solution was good (yellow).

Example 12

Neutralization of 75/25 Mixture of 5-Chloro-2-Methyl-3-Isothiazolone and 2-Methyl-3-Isothiazolone Using a Weak Base Ion Exchange Resin in Methanol

In a manner similar to Step 1 of Example 1, an isothiazolone•HCl salt was obtained and subjected to neutralization. Diaion® WA-30 ion exchange resin was used to neutralize the isothiazolone•HCl salt solution in methanol using the batch mode described in general procedure/method 1B. The yield of solvent-stripped isothiazolone free base was 98% with an unchanged isothiazolone mixture ratio, the purity of stripped product was 96%, and its color quality was very good (pale yellow).

Example 13

Neutralization of 75/25 Mixture of 5-Chloro-2-Methyl-3-Isothiazolone and 2-Methyl-3-Isothiazolone Using a Strong Base Ion Exchange Resin in Water

In a manner similar to Step 1 of Example 1, an isothiazolone•HCl salt was obtained and subjected to neutralization. Amberlite® IRA-420C-OH ion exchange resin was used to neutralize the isothiazolone•HCl salt solution in water using the batch mode described in general procedure/method 1A. The yield of isothiazolone free base was 87% and the color quality was poor (dark amber).

Example 14

Neutralization of 75/25 Mixture of 5-Chloro-2-Methyl-3-Isothiazolone and 2-Methyl-3-Isothiazolone Using a Strong Base Ion Exchange Resin in Methanol

In a manner similar to Step 1 of Example 1, an isothiazolone•HCl salt was obtained and subjected to neutralization. Amberlite® IRA-420C-OH ion exchange resin was used to neutralize the isothiazolone•HCl salt solution in methanol using the batch mode described in general procedure/method 1A. The yield of isothiazolone free base in the combined unstripped methanol effluent was 83%, the purity of the solvent-stripped product was 69%, and the color quality was very poor (amber).

Example 15

Neutralization of 75/25 Mixture of 5-Chloro-2-Methyl-3-Isothiazolone and 2-Methyl-3-Isothiazolone Using a Strong Base Ion Exchange Resin in Water

In a manner similar to Step 1 of Example 1, an isothiazolone•HCl salt was obtained and subjected to neutralization. Amberlite® IRA-420C-OH ion exchange resin was used to neutralize the isothiazolone•HCl salt solution in water using the column mode described in general procedure/method 2. The yield of isothiazolone free base was 79%, the isothiazolone mixture ratio had decreased significantly, and the color quality was very poor (dark amber).

Example 16

Neutralization of 75/25 Mixture of 5-Chloro-2-Methyl-3-Isothiazolone and 2-Methyl-3-Isothiazolone Using a Strong Base Ion Exchange Resin in Methanol

In a manner similar to Step 1 of Example 1, an isothiazolone•HCl salt was obtained and subjected to neutralization. Amberlite® IRA-420C-OH ion exchange resin was used to neutralize the isothiazolone•HCl salt solution in methanol using the column mode described in general procedure/method 2. The yield of unstripped isothiazolone free base was 100% and the color quality of the combined methanol effluent was good (yellow).

Example 17

Neutralization of 75/25 Mixture of 5-Chloro-2-Methyl-3-Isothiazolone and 2-Methyl-3-Isothiazolone Using a Strong Base Ion Exchange Resin in Water

In a manner similar to Step 1 of Example 1, an isothiazolone•HCl salt was obtained and subjected to neutralization. Amberlite® IRA-900C-OH ion exchange resin was used to neutralize the isothiazolone•HCl salt solution in water using the batch mode described in general procedure/method 1A. The yield of isothiazolone free base was 88%, the isothiazolone mixture ratio remained unchanged and the color quality of the unstripped free base isothiazolone aqueous effluent was poor (amber).

Example 18

Neutralization of 75/25 Mixture of 5-Chloro-2-Methyl-3-Isothiazolone and 2-Methyl-3-Isothiazolone Using a Strong Base Ion Exchange Resin in Methanol

In a manner similar to Step 1 of Example 1, an isothiazolone•HCl salt was obtained and subjected to neutralization. Amberlite® IRA-900C-OH ion exchange resin was used to neutralize the isothiazolone•HCl salt solution in methanol using the batch mode described in general procedure/method 1A. The yield of isothiazolone free base in the combined methanol effluent was 93%, the purity of the methanol-stripped product was only 45%, the isothiazolone mixture ratio was signficantly reduced and its color quality was very poor (dark amber).

Example 19

Neutralization of 75/25 Mixture of 5-Chloro-2-Methyl-3-Isothiazolone and 2-Methyl-3-Isothiazolone Using a Strong Base Ion Exchange Resin in Water

In a manner similar to Step 1 of Example 1, an isothiazolone•HCl salt was obtained and subjected to neutralization. Amberlite® IRA-900C-OH ion exchange resin was used to neutralize the isothiazolone•HCl salt solution in water using the column mode described in general procedure/method 2. The yield of isothiazolone free base was only 47%, the isothiazolone mixture ratio was significantly lower and the color quality was very poor (dark amber).

Example 20

Neutralization of 75/25 Mixture of 5-Chloro-2-Methyl-3-Isothiazolone and 2-Methyl-3-Isothiazolone Using a Strong Base Ion Exchange Resin in Methanol

In a manner similar to Step 1 of Example 1, an isothiazolone•HCl salt was obtained and subjected to neutralization. Amberlite® IRA-900C-OH ion exchange resin was used to neutralize the isothiazolone•HCl salt solution in methanol using the column mode described in general procedure/method 2. The yield of isothiazolone free base in methanol effluent was 97% with an unchanged isothiazolone mixture ratio, and the color quality of the unstripped free base isothiazolone methanol effluent was good (yellow).

## Claims

1. A process for preparing a 3-isothiazolone of the formula

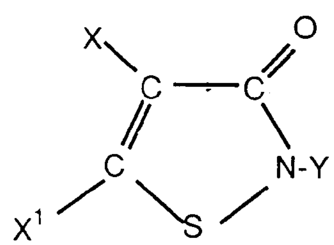

wherein
Y is an unsubstituted or hydroxy- or halogen-substituted alkyl of 1 to 10 carbon atoms; an unsubstituted or halogen-substituted alkenyl or alkynyl of 2 to 10 carbon atoms; or an aralkyl of up to 10 carbon atoms optionally substituted with halogen, lower alkyl, or lower alkoxy;
X is hydrogen or a ($C_1$-$C_2$) alkyl; and
$X^1$ is hydrogen, chlorine, or a ($C_1$-$C_2$) alkyl;
comprising
(a) preparing a solution of isothiazolone acid salt of the formula

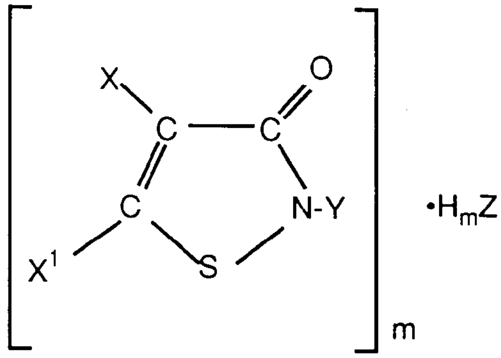

wherein

Z is chloride, bromide, sulfate, fluorosulfonate or trifluoromethyl sulfonate;

m is 1 when Z is chloride, bromide, fluorosulfonate, or trifluoromethyl sulfonate and m is 2 when Z is sulfate;

(b) treating said solution with a basic anion exchange resin; and

(c) separating the resultant free base isothiazolone solution from the resin.

2. Process according to claim 1 wherein m is 1 and Z is chloride or bromide.

3. The process according to Claim 1 or 2 wherein the solvent for the solution of isothiazolone salt is water, a $(C_1-C_5)$alkyl alcohol or a $(C_2-C_4)$alkylene glycol, preferably methanol, ethanol, isopropanol, ethylene glycol, propylene glycol, 1,3-butanediol, dipropylene glycol or diethylene glycol.

4. Process according to any preceding claim wherein the anion exchange resin contains a weakly basic functional group having a $pk_b$ value between 3 and 7, preferably between 3.5 and 4.5.

5. Process according claim 4, wherein the anion exchange resin is of the form

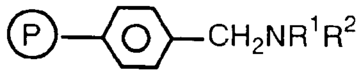 Resin C

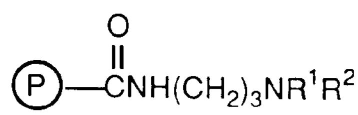 Resin D

or 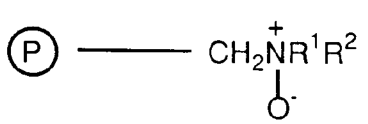 Resin E

wherein $R^1$ and $R^2$ are hydrogen, $(C_1-C_4)$alkyl, $(CH_2CH_2NH)_nH$ where n = 1-3, or β-hydroxy$(C_1-C_4)$alkyl; preferably methyl.

6. Process according to any preceding claim, wherein step (b) is performed in a column mode.

7. Process according to any of claims 1 to 5, wherein step (b) is performed in a batch mode.

8. Process according to any preceding claim, wherein the Y substituent on the isothiazolone is $(C_1-C_8)$alkyl, preferably n-octyl, and X and $X^1$ are both H.

9. Process according to any of claims 1 to 6 wherein Y is methyl, $X^1$ is hvyrogen or chlorine and X is hydrogen.

10. Process according to claim 9 wherein said resultant free base isothiazolone solution comprises a mixture of 5-chloro-2-methyl-3-isothiazolone and 2-methyl-3-isothiazolone, preferably in a ratio of from 92:8 to

3:97.

**11.** Process according to any preceding claim wherein said resultant free base isothiazolone solution is substantially free of water.

European Patent Office

# EUROPEAN SEARCH REPORT

Application Number

EP 91 31 1268

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 095 907 (ROHM AND HAAS COMPANY) * page 2, line 26 - page 8, line 14; claims * --- | 1 | C07D275/03 |
| A | EP-A-0 271 761 (RIEDEL-DE HAEN AKTIENGESELLSCHAFT) * the whole document * --- | 1 | |
| D,P, X | EP-A-0 437 354 (ROHM AND HAAS LTD) * claims * ----- | 1-11 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 27 FEBRUARY 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)